# EUROPEAN PATENT APPLICATION

(11) **EP 4 134 068 A1**
(43) Date of publication of application: **15.02.2023**
(21) Application number: 20929900.7
(22) Date of filing: 09.04.2020
(51) Int. Cl.: A61K 9/08, A61K 9/19, A61K 31/4184, A61K 47/40, A61P 35/00, A61P 35/02

(54) **BENDAMUSTINE COMPOSITION AND USE THEREOF**

(71) Applicant: Bika Biotechnology (Guangzhou) Co., Ltd., Guangzhou, Guangdong 510663 (CN)
(72) Inventor: JING, Yue, Nanjing, Jiangsu 210038 (CN); YAN, Qing, Nanjing, Jiangsu 210038 (CN)
(74) Representative: Pharma Patents International AG
(86) International application number: PCT/CN2020/084038
(87) International publication number: WO 2021/203377

(57) **Abstract**

Provided is a composition comprising the following components: a) bendamustine or a salt thereof, b) a cyclodextrin, and c) an optional stabilizer, wherein the stabilizer is selected from an inorganic or organic substance containing chlorine element, lipoic acid, thioglycerol, polyvinylpyrrolidone and any combination thereof, and wherein the mass ratio of the bendamustine or the salt thereof to the cyclodextrin is 1 : 5 to 1 : 100. Further provided is a method for preventing or treating a cancer using the composition and the use of the composition in the preparation a medicament for treating a cancer.

## Description

### Technical Field

The present invention relates to a composition comprising bendamustine and a salt thereof, a cyclodextrin and an optional stabilizer and the use thereof.

### Background Art

Bendamustine is used for treating various cancers, including leukemia, Hodgkin's disease and multiple myeloma. It is required currently in the product instructions of the trade name Treanda^{™} to dilute a reconstituted product (within 30 minutes) into 500 ml of a diluent (normal saline, and 2.5% glucose/0.45% saline) for infusion, with the infusion at 100 mg/m² for 30 minutes or at 120 mg/m² for 60 minutes. A diluted mixture of Balrapzo and Treanda can be stored at 2-8ºC for 24 hours and at room temperature (15-30ºC) for 3 hours. Since bendamustine is highly unstable in water, the infusion must be completed within 3 hours after being compatible with normal saline and 2.5% glucose/0.45% saline.

A currently approved freeze-dried bendamustine powder (such as Treanda) requires a reconstitution time of 15-30 minutes, and the reconstitution process is accompanied by decomposition. After reconstitution, it needs to be diluted in time and then infused to a patient within 3 hours for completion.

Although ready-to-use bendamustine (such as Bendeka) does not need to be reconstituted, propylene glycol is used which reduces the formation of a PEG ester. There are far more impurity varieties than in Treanda, and propylene glycol has certain potential risks, which may lead to renal failure and arrhythmia.

In addition, for patients who need to be treated with bendamustine, if the patients are suffering from diseases such as a serious heart disease, acute left heart failure, congestive heart failure, renal failure, liver cirrhosis, ascites, cerebral edema, idiopathic edema, hypertension and hypokalemia, the use of normal saline should be avoided as much as possible. However, existing commercially available products have a content of less than 95% one hour after dilution with 5% glucose, so they are clinically unavailable. After a 0.9% normal saline is prepared, the time from preparation to infusion shall not exceed 3 hours. In addition, 2.5% glucose/0.45% saline is not commonly used for infusion. When a hospital is out of stock, a bottle of 250 mL 5% glucose and a bottle of 250 mL 0.9% normal saline are often mixed and injected into a 500 mL infusion bag, which increases the risk of microbial contamination and the preparation time. Moreover, the 2.5% glucose/0.45% saline also merely reduces the burden of sodium chloride of a patient by half.

In addition, for some patients who need to control the sugar intake, such as patients with diabetes, or patients who need to control both the salt and sugar intakes, such as patients with three-high syndrome, bendamustine injections prepared with 0.45% saline, 2.5% glucose/0.45% saline, 2.5% glucose and even water for injection can better eliminate the risks and worries of patients. In view of the above-mentioned problems, the present invention aims to provide a bendamustine preparation which can be diluted to 0.2-100 mg/mL, while satisfying the needs of patients with low salt, low sugar, salt-free and sugar-free requirements, and can be stable for a long time after dilution, thereby reducing the risk caused by rapidly increasing impurities, improving the convenience of clinical medication and reducing the amount of abandoned drugs, which will be of great significance, this also being exactly a goal of the development of the preparation provided by the present invention.

### Summary of the Invention

It has always been known that bendamustine or a salt thereof (API) cannot be compatible with 5% glucose. If a patient needs to reduce the intake of sodium chloride, only a 2.5% glucose/0.45% sodium chloride solution can be used for infusion. Through research, we have unexpectedly found a composition comprising bendamustine or a salt thereof, which composition can be stabilized for not less than 3 hours after being diluted with 5% glucose, thereby providing a hope of treatment with bendamustine or a salt thereof for patients who cannot intake too much sodium chloride.

The present invention mainly provides a composition comprising bendamustine or a salt thereof, which composition can provide stability (the degradation of API is not more than 5 wt%) for at least 3 hours at room temperature (15-30ºC) and under natural light condition when diluted with a 5% glucose solution to an API concentration of 0.2 mg/mL. As described herein, "stability" refers to the time required for the composition comprising bendamustine or a salt thereof to degrade by 5 wt% after reconstitution or dilution. It can be understood that the longer the time required for bendamustine or a salt thereof to degrade by 5 wt% or the less the bendamustine or the salt thereof degraded within a specified time, the more stable a reconstituted solution or diluted solution of bendamustine or the salt thereof. The stability herein includes cold storage stability and room temperature stability, wherein cold storage stability refers to stability at 2-8ºC and room temperature stability refers to stability at 15-30ºC.

The present invention provides a composition comprising bendamustine or a salt thereof, which composition can provide stability (the degradation of API is not more than 5 wt%) for at least 6 hours at room temperature (15-30ºC) and under natural light condition when diluted with a 0.9% sodium chloride solution to an API concentration of 0.2 mg/mL.

The present invention provides a composition comprising bendamustine or a salt thereof, which composition can be reconstituted and diluted with water for injection, 0.9% sodium chloride, 0.45% sodium chloride 2.5% glucose/0.45% sodium chloride, 2.5% glucose and 5% glucose.

The present invention provides a composition comprising bendamustine or a salt thereof, which composition can provide stability (the degradation of API is not more than 5 wt%) for at least 3 hours at room temperature (15-30ºC) and under natural light condition when reconstituted and diluted with water for injection, 0.9% sodium chloride, 0.45% sodium chloride 2.5% glucose/0.45% sodium chloride, 2.5% glucose and 5% glucose to an API concentration of more than 0.2 mg/mL, e.g. 0.6, 1.0, 2.5, 5, 10, 16, 25 or 100 mg/mL. The values of 0.2 mg/mL etc. are not fixed. The present composition can be diluted to any concentration of API in the range of 0.2-100 mg/mL, none of which can result in precipitation caused by solubility limitation, and which can also all provide stability for at least 3 hours at room temperature (15-30ºC) and under natural light condition (the degradation of API is not more than 5 wt%).

In the study, we have also found that the presence of sodium chloride can reduce the solubility of bendamustine, which makes it impossible to obtain a smaller infusion volume. The composition of the present invention makes bendamustine within a concentration of 0.2-100 mg/mL while allowing for no precipitation caused by insufficient solubility; therefore, the need of a patient for a smaller infusion volume can be satisfied, and the composition of the present invention can be diluted to 1-500 mL for use by the patient. For example, according to a dosage of 100 mg/m², a patient with a BSA of 1 needs 100 mg of bendamustine, and the composition of the present invention can be diluted with a diluent to 1 mL to obtain a 100 mg/mL drug solution, and can be diluted to 4 mL to obtain a 25 mg/mL drug solution, to 10 mL to obtain a 10 mg/mL drug solution, to 20 mL to obtain 5 mg/mL drug solution, to 50 mL to obtain a 2 mg/mL drug solution, to 100 mL to obtain a 1 mg/mL drug solution, to 250 mL to obtain a 0.4 mg/mL drug solution or to 500 mL to obtain a 0.2 mg/mL drug solution. The diluent can be water for injection, normal saline, 5% glucose, 2.5% glucose, 0.45% sodium chloride, 0.45% sodium chloride/2.5% glucose, etc. There is no crystal precipitation from the diluted drug solutions, and an infusion time of not less than 3 hours is provided.

The present invention provides a composition comprising bendamustine or a salt thereof, which composition can be stored at 2-8ºC in a dark environment for at least 28 days (the degradation of API is not more than 5 wt%) after being reconstituted with water for injection and normal saline; in addition, a drug solution reconstituted on the first day of a treatment cycle can be stored until the 28th day of the treatment cycle, thereby reducing the economic loss of patients. After being reconstituted, commercially available freeze-dried powders cannot be stabilized at 2-8ºC for 28 days (the degradation of API is less than 5 wt%).

The bendamustine included in some embodiments of the present invention is bendamustine, or bendamustine hydrochloride, or another bendamustine salt.

In some embodiments of the present invention, the composition comprises a cyclodextrin. Although cyclodextrins can improve the solubility and stability of a poorly soluble drug by means of lipophilic cavities, there are few studies on improving the stability and solubility of water-soluble drugs.

Cyclodextrins are a wide variety of cyclic polysaccharides, which may be cylindrical supramolecular structures composed of 6 or 7 polycyclic rings, and they can be divided into methylated cyclodextrins, hydroxypropyl cyclodextrins, sulfobutyl cyclodextrins, etc., according to different derivatization groups, can be divided into α-cyclodextrins, β-cyclodextrins, γ-cyclodextrins, etc., according to the number of polycyclic rings, and can be divided into basic cyclodextrins, acidic cyclodextrins, neutral cyclodextrins, etc., according to pH.

During the research process, we have unexpectedly found that compositions of cyclodextrins of the same type and bendamustine at the same mass ratio exhibited inconsistency in stability at room temperature after dissolution. This seriously affects the clinical use safety of a bendamustine cyclodextrin inclusion preparation. After repeatedly comparing different types of cyclodextrins from different manufacturers and batches, we found that during the preparation of cyclodextrin derivatives, it was required to use sodium hydroxide to produce an alkaline environment, and sodium hydroxide and sodium chloride were then removed using means such as a hydrochloric acid or strong acid ion exchange resin.

What affects, to the greatest extent, the stability of bendamustine is a class of cyclodextrins containing a sodium hydroxide residue. The presence of sodium hydroxide makes the pH of cyclodextrin products alkaline, causing bendamustine and a salt thereof to degrade in an accelerated manner, resulting in an abnormal increase of impurities.

The residual sodium hydroxide can be removed by means of cation exchange to obtain a cyclodextrin free of sodium hydroxide and/or sodium chloride. Such cyclodextrins result in a relatively poor stability of a composition with bendamustine based on the same weight ratio, which is more significant particularly when it is compatible with glucose.

A chlorine-containing cyclodextrin is formed by neutralizing the residual sodium hydroxide to form sodium chloride, and with or without desalting, the final chloride ion content is in the range of 0.001-5 wt%. Bendamustine is more stable than in combination with the same mass of a desalted cyclodextrin, and the stability of bendamustine increases with the increase of the chloride ion content However, cyclodextrins of different manufacturers, different processes, different batches and different types have relatively great differences in chloride ion residues. Different sodium chloride removal processes can make the content of chloride ions in the cyclodextrins float within 0-5% (w/w%), and one gram of cyclodextrin carries about 0-50 mg of sodium chloride. The larger the mass ratio of cyclodextrin to bendamustine, the larger the mass ratio of chloride ions to bendamustine. In a bendamustine preparation, chloride ions act as a stabilizer, and an excessively high chloride ion content not only threatens the life and health of patients with a low sodium diet, but is also disadvantageous for complex product quality which causes potential quality hazards. The addition of the stabilizer can reduce the amount of the cyclodextrin and reduce the risk of use by patients with impaired renal function. If the content of chlorine ions in the cyclodextrin is rationally controlled and the amount of the stabilizer is adjusted, a bendamustine preparation with a better stability and a uniform quality can be obtained.

In the present patent, a cyclodextrin with a chloride ion content of 0-5% (w/w) is used to improve the stability of the bendamustine and the salt thereof, and preferably, a cyclodextrin with a chloride ion content of 0-3% (w/w), and further preferably a chloride ion content of 0-1% (w/w), e.g. 0.01-1% (w/w), is used to improve the stability of the bendamustine and the salt thereof. As used herein, the chlorine-containing cyclodextrin refers to a cyclodextrin containing sodium chloride, wherein the content of chlorine in the cyclodextrin refers to the mass percentage of sodium chloride relative to the cyclodextrin.

The cyclodextrin in some embodiments of the present invention is sulfobutyl-β-cyclodextri n. The degree of substitution of the sulfobutyl-β-cyclodextrin may be 1-11, and a degree of su bstitution of 6-7 is the most widely used.

The cyclodextrin in some embodiments of the present invention is hydroxypropyl-β-cyclod extrin. The degree of substitution of the hydroxypropyl-β-cyclodextrin may be 1-11, and a deg ree of substitution of 4-9 is the most widely used.

In some more preferred embodiments, the cyclodextrin is sulfobutyl-β-cyclodextrin.

In some embodiments of the present invention, the stabilizer includes inorganic or organic substances containing chlorine element, such as sodium chloride and choline chloride, and the stabilizer helps to cooperate with the cyclodextrin in order to provide a composition no worse than that containing the cyclodextrin only.

In some embodiments of the present invention, the stabilizer includes polyvinylpyrrolidone (e.g., PVPK12, PVPK17, and PVPK30), lipoic acid, thioglycerol, etc., and the stabilizer helps to cooperate with the cyclodextrin in order to provide a composition no worse than that containing the cyclodextrin only.

By adjusting the content of chlorine and the content of the stabilizer in the cyclodextrin, the present invention can inhibit the degradation of bendamustine and the salt thereof and shorten the time for the dissolution of bendamustine and the salt thereof, thereby achieving a great advantage of use of bendamustine in a low sodium environment.

In the present invention, a bacteriostatic agent, an osmotic pressure regulator, etc. may be further added according to known schemes.

In the present invention, a lyoprotectant or a proppant, such as mannitol, may be further added according to known schemes. The lyoprotectant or proppant is used for changing the morphology and re-dissolution time of a freeze-dried powder.

The mass ratio of the bendamustine and the salt thereof to the cyclodextrin in the present invention is 1 : 5-100. Although a higher proportion of the cyclodextrin can provide a better stability, problems, e.g. costs and auxiliary material safety, under realistic conditions exist. All individual values and subranges of the mass ratio of 1 : 5-100 are included and disclosed herein. For example, the mass ratio of the bendamustine and the salt thereof to the cyclodextrin may be 1 : 5-24, or may also be 1 : 24-100.

The mass ratio of the bendamustine and the salt thereof to the stabilizer in the present invention is 1 : 0-5. All individual values and subranges of the mass ratio of 1 : 0-5 are included and disclosed herein.

The composition of the present invention can be prepared into a dry powder preparation by means of freeze drying and spray drying. The concentration of bendamustine in a solution before freeze-drying may be 0.2-100 mg/mL. All individual values and subranges of 0.2-100 mg/mL are included and disclosed herein.

In addition, the present invention provides a composition or injection comprising the above-mentioned diluent, which composition or injection comprises bendamustine or a salt thereof, a cyclodextrin, an optional stabilizer, and a diluent, wherein the mass ratio of the bendamustine or the salt thereof to the cyclodextrin is 1 : 5 to 1 : 100, and the diluent is selected from the group consisting of water for injection, 0.9% sodium chloride, 0.45% sodium chloride, 0.45% sodium chloride/2.5% glucose, 2.5% glucose and 5% glucose. In some embodiments, the diluent is 5% glucose.

The present invention further provides a method for preventing or treating a cancer, the method comprising providing a subject with an injection of bendamustine or a salt thereof at a concentration of 0.2-100 mg/ml, wherein the injection comprises the bendamustine or the salt thereof, a cyclodextrin, an optional stabilizer and a diluent, with the mass ratio of the bendamustine or the salt thereof to the cyclodextrin being 1 : 5-100.

The present invention further provides the use of the composition according to the present invention in the preparation a medicament for treating a cancer. The cancer is selected from the group consisting of chronic lymphocytic leukemia, Hodgkin's lymphoma, non-Hodgkin's lymphoma, multiple myeloma and breast cancer.

Unless otherwise defined, all technical and scientific terms as used herein have the same meanings as commonly understood by a person of ordinary skill in the art

### Brief Description of the Drawings

Figure 1 shows a variation curve of the percentage of the API content over the time t (hour) in Example 1.
Figure 2 shows an X-ray powder diffraction pattern of a freeze-dried powder in Example 16.

### Examples

The following examples are used to further illustrate the present invention, but do not limit the effective scope of the present invention in any way.

For the content determination by HPLC and related substances in the examples, reference can be made to USP43 draft of the United States Pharmacopoeia with regard to raw bendamustine material and preparations. The HPLC method is as follows:

| | | | |
|---|---|---|---|
| Chromatographic column | L60, 150 mm × 4.6 mm, 5 µm | | |
| Detection wavelength | 254 nm | | |
| Column temperature | 30ºC | | |
| Flow rate | 1.0 mL/min | | |
| Injection volume | 20 uL | | |
| Mobile phase A | 0.1% (V/V) trifluoroacetic acid-water | | |
| Mobile phase B | 0.1% (V/V) trifluoroacetic acid-acetonitrile | | |

| Gradient elution program | Time (minutes) | Mobile phase A | Mobile phase B (%) |
|---|---|---|---|
| | 0 | 93 | 7 |
| | 5 | 93 | 7 |
| | 13 | 73 | 27 |
| | 16 | 73 | 27 |
| | 25 | 43 | 57 |
| | 26 | 10 | 90 |
| | 31 | 10 | 90 |
| | 40 | 93 | 7 |
| | 45 | 93 | 7 |

Unless otherwise specified, room temperature in the examples refers to 15-30ºC.

Unless otherwise specified, cold storage in the examples refers to 2-8ºC.

Freeze drying in the examples can also be replaced by another drying means such as spray drying.

In the examples, a 0.9% sodium chloride injection may also be normal saline, and a 0.45% sodium chloride/2.5% glucose injection is also expressed as equivalent descriptions such as a 2.5% glucose/0.45% sodium chloride injection or a half-salt and half-sugar injection.

As used in the examples, the term "dry powder" or "dry powder preparation" refers to any solid material obtained by the continuous drying, such as spray drying or fluidized bed drying, of a non-aqueous solution or an aqueous solution or a combination of an aqueous solution and a non-aqueous solution. In the following examples, those where no specific conditions are indicated should all be carried out according to conventional conditions in the art or conditions suggested by manufacturers.

The API shown in the examples is bendamustine hydrochloride.

### Example 1

### Stability of API in composition placed at room temperature 48 hours after being diluted to 0.2 mg/mL with 0.9% sodium chloride

| Component (each) | | API: Cyclodextrin |
|---|---|---|
| API | 100 mg | 1 : 8 |
| Sulfobutyl-β-cyclodextrin | 800 mg | |
| Water for injection (for dilution to a constant volume) | 2 mL | |

800 mg of sulfobutyl-β-cyclodextrin was dissolved with water for injection (the content of chlorine in sulfobutyl-β-cyclodextrin was 0.08 wt%) and diluted to a constant volume of 2 mL, the solution was then cooled to 4ºC, the API was added and stirred until completely dissolved over about 5 minutes, and the solution was filtered with a 0.22 µm filter, diluted to 0.2 mg/mL with 0.9% sodium chloride, left to stand at room temperature, and determined for 48-hour stability by means of HPLC. The data was as shown in the following table:

| **Time (h)** | **API (C/C0%)** |
|---|---|
| **0** | 100.00 |
| **4** | 97.77 |
| **8** | 95.67 |
| **12** | 93.59 |
| **16** | 91.65 |
| **20** | 89.77 |
| **24** | 87.86 |
| **28** | 86.04 |
| **32** | 84.27 |
| **36** | 82.55 |
| **40** | 80.85 |
| **44** | 79.27 |
| **48** | 77.64 |

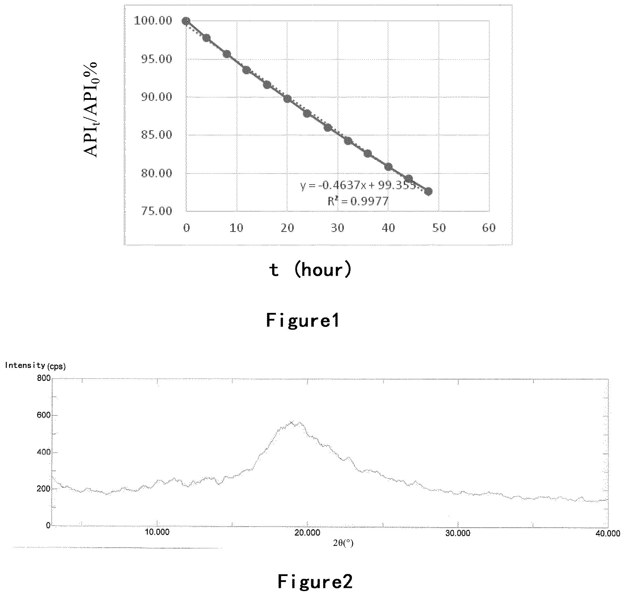

According to the above-mentioned data, with time as abscissa and API percentage as ordinate (see attached figure 1), it could be seen that the decrease of the API content was linear, the degradation rate was not obviously affected by the cyclodextrin, and the action of the combination of the API and the cyclodextrin did not affect the drug release.

### Example 2

**Influence of stirring temperature on impurities in solution before freeze-drying**

| | **Component (each)** | | **Stirring temperature** |
|---|---|---|---|
| **1** | API | 100 mg | 4ºC |
| | Sulfobutyl-β-cyclodextrin | 2400 mg | |
| | Water for injection (for dilution to a constant volume) | 4 mL | |
| **2** | API | 100 mg | 10ºC |
| | Sulfobutyl-β-cyclodextrin | 2400 mg | |
| | Water for injection (for dilution to a constant volume) | 4 mL | |
| **3** | API | 100 mg | 20ºC |
| | Sulfobutyl-β-cyclodextrin | 2400mg | |
| | Water for injection (for dilution to a constant volume) | 4 mL | |

2400 mg of sulfobutyl-β-cyclodextrin was dissolved with water for injection (the content of chlorine in sulfobutyl-β-cyclodextrin was 0.08 wt%) and diluted to a constant volume of 4 mL, the solutions were then cooled to respectively 4ºC, 10ºC and 20ºC, the API was added and stirred until completely dissolved over about 5 minutes, the stirring continued for 30 minutes, the solutions were filtered with a 0.22 µm filter, diluted to 0.2 mg/mL with 0.9% sodium chloride, and measured for the content thereof and impurities at 0 h by means of HPLC. The data was as shown in the following table:

| **Temperature (ºC)** | **HP1^{∗} (area%)** | **API (area%)** |
|---|---|---|
| **4** | 0.22 | 99.59 |
| **10** | 0.21 | 99.60 |
| **20** | 0.23 | 99.56 |

Note: HP1 impurity was the most predominant degradation impurity from bendamustine, and the short stable time of bendamustine in the aqueous solution was mainly due to the HP1 produced by hydrolysis. The structural formula of HP1 was as follows:

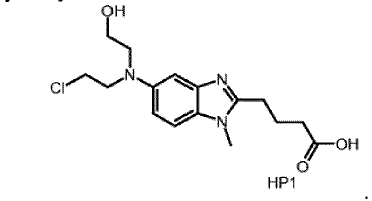

It could be seen from the data in the above table that sulfobutyl-β-cyclodextrin made it possible to prepare bendamustine at a relatively high temperature, and it was not necessary to prepare bendamustine at 4-15ºC according to an existing preparation process.

### Example 3

### Stability of composition after being diluted with different dilution solvents

| | **Component (each)** | | **Stirring temperature** |
|---|---|---|---|
| **1** | API | 100 mg | 4ºC |
| | Sulfobutyl-β-cyclodextrin | 2400 mg | |
| | Water for injection (for dilution to a constant volume) | 4 mL | |

2400 mg of sulfobutyl-β-cyclodextrin was dissolved with water for injection (the content of chlorine in sulfobutyl-β-cyclodextrin was 0.08 wt%) and diluted to a constant volume of 4 mL, the API was added and stirred until completely dissolved over about 5 minutes, the stirring continued for 30 minutes, and the solution was filtered with a 0.22 µm filter, diluted to 0.2 mg/mL with a corresponding dilution solvent, and determined for 24-hour cold storage stability and 6-hour room temperature stability by means of HPLC. The data was as shown in the following table:

**24-hour cold storage stability**

| | Time (h) | HP1 (area%) | API (area%) | API (C/C0%) |
|---|---|---|---|---|
| **5% glucose** | 0 | 0.23 | 99.77 | 100.00 |
| | 6 | 0.41 | 99.59 | 100.00 |
| | 12 | 0.58 | 99.37 | 99.50 |
| | 24 | 0.93 | 99.00 | 100.00 |
| **0.9% sodium chloride** | 0 | 0.18 | 99.82 | 100.00 |
| | 6 | 0.28 | 99.72 | 100.00 |
| | 12 | 0.37 | 99.63 | 99.50 |
| | 24 | 0.55 | 99.45 | 99.50 |
| **Water for injection** | 0 | 0.22 | 99.78 | 100.00 |
| | 6 | 0.41 | 99.59 | 100.00 |
| | 12 | 0.62 | 99.38 | 99.50 |
| | 24 | 1.01 | 98.89 | 99.00 |
| **2.5% glucose** | 0 | 0.23 | 99.77 | 100.00 |
| | 6 | 0.41 | 99.59 | 99.51 |
| | 12 | 0.59 | 99.41 | 99.51 |
| | 24 | 0.94 | 99.01 | 98.52 |
| **0.45% sodium chloride** | 0 | 0.21 | 99.79 | 100.00 |
| | 6 | 0.31 | 99.69 | 99.50 |
| | 12 | 0.42 | 99.58 | 100.00 |
| | 24 | 0.64 | 99.36 | 99.50 |
| **2.5% glucose/0.45% sodium chloride** | 0 | 0.22 | 99.78 | 100.00 |
| | 6 | 0.33 | 99.67 | 99.50 |
| | 12 | 0.42 | 99.58 | 99.50 |
| | 24 | 0.64 | 99.36 | 99.50 |

It could be seen from the above table that after dilution to 0.2 mg/mL with 0.9% sodium chloride or 5% glucose and placement under cold storage for 24 hours, the API content had almost no change and the stability of the composition was greatly improved.

### 6-hour cold storage stability

| | Time (h) | HP1 (area%) | API (area%) | API (C/C0%) |
|---|---|---|---|---|
| **0.9% sodium chloride** | 0 | 0.23 | 99.77 | 100.00 |
| | 3 | 1.54 | 98.39 | 98.13 |
| | 6 | 2.76 | 97.07 | 96.73 |
| **5% glucose** | 0 | 0.24 | 99.73 | 100 |
| | 3 | 2.48 | 97.27 | 97.13 |
| | 6 | 4.40 | 95.04 | 94.74 |
| **Water for injection** | 0 | 0.23 | 99.72 | 100.00 |
| | 3 | 3.15 | 96.59 | 96.02 |
| | 6 | 5.55 | 93.69 | 92.54 |
| **2.5% glucose** | 0 | 0.34 | 99.46 | 100 |
| | 3 | 3.15 | 96.47 | 96.67 |
| | 6 | 5.37 | 93.97 | 93.85 |
| **0.45% sodium chloride** | 0 | 0.23 | 99.75 | 100.00 |
| | 3 | 1.99 | 97.93 | 98.00 |
| | 6 | 3.61 | 96.14 | 96.00 |
| **2.5% glucose/0.45% sodium chloride** | 0 | 0.22 | 99.74 | 100.00 |
| | 3 | 1.84 | 98.03 | 97.52 |
| | 6 | 3.33 | 96.39 | 95.54 |

It could be seen from the above table that after dilution to 0.2 mg/mL with water for injection, 5% glucose, 2.5% glucose, 2.5% glucose/0.45% sodium chloride, 0.45% sodium chloride and 0.9% sodium chloride, at least a stable time of not less than three hours could be provided (the degradation of API was less than 5%).

### Example 4

### Investigation on crystal precipitation at 4ºC of API in composition diluted to different concentrations with different dilution solvents

| | **Component (each)** | | **Stirring temperature** |
|---|---|---|---|
| **1** | API | 100 mg | 4ºC |
| | Sulfobutyl-β-cyclodextrin | 2400 mg | |
| | Water for injection (for dilution to a constant volume) | 4 mL | |

2400 mg of sulfobutyl-β-cyclodextrin was dissolved with water for injection (the content of chlorine in sulfobutyl-β-cyclodextrin was 0.08 wt%) and diluted to a constant volume of 4 mL, the API was added and stirred until completely dissolved over about 5 minutes, the stirring continued for 30 minutes, the solution was filtered with a 0.22 µm filter, diluted to 100 mg/mL with a corresponding dilution solvent, and placed under cold storage at 2-8ºC, and the crystal precipitation situation was observed at 24 h and day 28:

| | Concentration (mg/mL) | 24 h | 28 d |
|---|---|---|---|
| 0.9% sodium chloride | 0.2 | No crystal precipitation | No crystal precipitation |
| | 0.6 | No crystal precipitation | No crystal precipitation |
| | 50 | No crystal precipitation | No crystal precipitation |
| | 100 | No crystal precipitation | No crystal precipitation |
| 5% glucose | 0.2 | No crystal precipitation | No crystal precipitation |
| | 0.6 | No crystal precipitation | No crystal precipitation |
| | 50 | No crystal precipitation | No crystal precipitation |
| | 100 | No crystal precipitation | No crystal precipitation |
| Water for injection | 0.2 | No crystal precipitation | No crystal precipitation |
| | 0.6 | No crystal precipitation | No crystal precipitation |
| | 50 | No crystal precipitation | No crystal precipitation |
| | 100 | No crystal precipitation | No crystal precipitation |
| 2.5% glucose | 0.2 | No crystal precipitation | No crystal precipitation |
| | 0.6 | No crystal precipitation | No crystal precipitation |
| | 50 | No crystal precipitation | No crystal precipitation |
| | 100 | No crystal precipitation | No crystal precipitation |
| 0.45% sodium chloride | 0.2 | No crystal precipitation | No crystal precipitation |
| | 0.6 | No crystal precipitation | No crystal precipitation |
| | 50 | No crystal precipitation | No crystal precipitation |
| | 100 | No crystal precipitation | No crystal precipitation |
| 2.5% glucose/0.45% sodium chloride | 0.2 | No crystal precipitation | No crystal precipitation |
| | 0.6 | No crystal precipitation | No crystal precipitation |
| | 50 | No crystal precipitation | No crystal precipitation |
| | 100 | No crystal precipitation | No crystal precipitation |

It could be seen from the above table that bendamustine or the salt thereof in the composition could achieve a solubility of 0.2-100 mg/mL in various dilution solvents, and no crystal precipitation induced by a low solubility would occur when in clinical use.

### Example 5

### Stability of composition containing hydroxypropyl-β-cyclodextrin after being diluted with 5% glucose

| | Component (each) | | API : Cyclodextrin |
|---|---|---|---|
| **1** | API | 100 mg | 1 : 24 |
| | Hydroxypropyl-β-cyclodextrin | 2400 mg | |
| | Sodium chloride | 40 mg | |
| | Water for injection (for dilution to a constant volume) | 4 mL | |

A corresponding prescription amount of the cyclodextrin was dissolved with water for injection and diluted to a corresponding volume, the solution was cooled to 4ºC, the API was added and stirred until completely dissolved over about 5 minutes, and the solution was filtered with a 0.22 µm filter, diluted to 0.2 mg/mL with 5% glucose, left to stand at room temperature, and determined for 6-hour stability by means of HPLC. The data was as shown in the following table:

| | **Time (h)** | **HP1 (area%)** | **API (area%)** | **API (C/C0%)** |
|---|---|---|---|---|
| **1** | 0 | 0.24 | 99.57 | 100.00 |
| | 3 | 3.12 | 96.65 | 97.25 |
| | 6 | 6.00 | 93.66 | 93.81 |

It could be seen from the above table that hydroxypropyl-β-cyclodextrin (the content of chlorine in hydroxypropyl-β-cyclodextrin was 0.08 wt%) could be used for providing a composition with a stable time of not less than three hours (the degradation of API was less than 5%).

### Example 6

### Stability of compositions comprising API and sulfobutyl-β-cyclodextrin at different mass ratios after being diluted with 5% glucose

| | Component (each) | | API : Cyclodextrin |
|---|---|---|---|
| **1** | API | 100 mg | 1 : 5 |
| | Sulfobutyl-β-cyclodextrin | 500 mg | |
| | Water for injection (for dilution to a constant volume) | 4 mL | |
| **2** | API | 100 mg | 1 : 12 |
| | Sulfobutyl-β-cyclodextrin | 1200 mg | |
| | Water for injection (for dilution to a constant volume) | 4 mL | |
| **3** | API | 100 mg | 1 : 36 |
| | Sulfobutyl-β-cyclodextrin | 3600 mg | |
| | Water for injection (for dilution to a constant volume) | 6 mL | |
| **4** | API | 100 mg | 1 : 60 |
| | Sulfobutyl-β-cyclodextrin | 6000 mg | |
| | Water for injection (for dilution to a constant volume) | 10 mL | |
| **5** | API | 24 mg | 1:100 |
| | Sulfobutyl-β-cyclodextrin | 2400 mg | |
| | Water for injection (for dilution to a constant volume) | 4 mL | |

A corresponding prescription amount of sulfobutyl-β-cyclodextrin was dissolved with water for injection (the content of chlorine in sulfobutyl-β-cyclodextrin was 0.08 wt%) and diluted to a corresponding volume, the solution was then cooled to 4ºC, the API was added and stirred until completely dissolved over about 5 minutes, and the solution was filtered with a 0.22 µm filter, diluted to 0.2 mg/mL with a 5% glucose injection, left to stand at room temperature, and determined for 6-hour stability by means of HPLC. The data was as shown in the following table:

| | **Time (h)** | **HP1 (area%)** | **API (area%)** | **API (C/CO%)** |
|---|---|---|---|---|
| **1** | 0 | 0.28 | 99.7 | 100.00 |
| | 3 | 5.33 | 94.09 | 93.15 |
| | 6 | 9.34 | 89.05 | 87.10 |
| **2** | 0 | 0.28 | 99.72 | 100.00 |
| | 3 | 3.71 | 95.87 | 95.23 |
| | 6 | 6.50 | 92.42 | 90.95 |
| **3** | 0 | 0.20 | 99.80 | 100.00 |
| | 3 | 2.07 | 97.69 | 97.81 |
| | 6 | 3.67 | 95.84 | 94.89 |
| **4** | 0 | 0.23 | 99.77 | 100.00 |
| | 3 | 1.87 | 97.93 | 97.65 |
| | 6 | 3.33 | 96.23 | 95.29 |
| **5** | 0 | 0.20 | 99.78 | 100.00 |
| | 3 | 1.43 | 98.47 | 98.08 |
| | 6 | 2.26 | 97.55 | 96.15 |

It could be seen from the above table that with the increase of the proportion of the cyclodextrin, the degradation rate of the API became smaller and smaller after the API in the composition was diluted to 0.2 mg/mL with 5% glucose.

### Example 7

### Stability of compositions containing sodium chloride after being diluted with 5% glucose

| Component (each) | | API : sodium chloride |
|---|---|---|
| API | 100 mg | 1 : 0.36 |
| Sulfobutyl-β-cyclodextrin | 2400 mg | |
| Sodium chloride | 36 mg | |
| Water for injection (for dilution to a constant volume) | 4 mL | |
| API | 100 mg | 1 : 0.72 |
| Sulfobutyl-β-cyclodextrin | 2400 mg | |
| Sodium chloride | 72 mg | |
| Water for injection (for dilution to a constant volume) | 4 mL | |

Corresponding prescription amounts of sulfobutyl-β-cyclodextrin and sodium chloride were dissolved with water for injection (the content of chlorine in sulfobutyl-β-cyclodextrin was 0.08 wt%) and diluted to a corresponding volume, the solution was then cooled to 4ºC, the API was added and stirred until completely dissolved over about 30-120 minutes, and the solution was filtered with a 0.22 µm filter, diluted to 0.2 mg/mL with a 5% glucose injection, left to stand at room temperature, and determined for 6-hour stability by means of HPLC. The data was as shown in the following table:

| | **Time (h)** | **HP1 (area%)** | **API (area%)** | **API (C/C0%)** |
|---|---|---|---|---|
| **1** | 0 | 0.17 | 99.82 | 100.00 |
| | 3 | 1.74 | 98.19 | 97.63 |
| | 6 | 3.21 | 96.43 | 95.73 |
| **2** | 0 | 0.19 | 99.80 | 100.00 |
| | 3 | 1.68 | 98.23 | 98.11 |
| | 6 | 3.12 | 96.51 | 95.75 |

The time for the dissolution of the prescription in which sodium chloride was added was longer than that of the composition in which no sodium chloride was added. The time for the dissolution of the prescription in which no sodium chloride was added was about 5 minutes, and the time for the dissolution of the prescription in which sodium chloride was added varied from 5 to 120 minutes.

### Example 8

### Stability of compositions containing cyclodextrins with different chlorine contents after being diluted with 5% glucose

| | **Component (each)** | | **API: Cyclodextrin** |
|---|---|---|---|
| **1** | API | 100 mg | 1 : 24 |
| | Sulfobutyl-β-cyclodextrin a | 2400 mg | |
| | Water for injection | 4 mL | |
| **2** | API | 100 mg | 1 : 24 |
| | Sulfobutyl-β-cyclodextrin b | 2400 mg | |
| | Water for injection | 4 mL | |
| **3** | API | 100 mg | 1 : 24 |
| | Sulfobutyl-β-cyclodextrin c | 2400 mg | |
| | Water for injection | 4 mL | |
| **4** | API | 100 mg | 1 : 24 |
| | Sulfobutyl-β-cyclodextrin d | 2400 mg | |
| | Water for injection | 4 mL | |

The content of chlorine in sulfobutyl-β-cyclodextrin a was below a detection line and was regarded as 0;
the content of chlorine in sulfobutyl-β-cyclodextrin b was 0.01%;
the content of chlorine in sulfobutyl-β-cyclodextrin c was 1%; and
the content of chlorine in sulfobutyl-β-cyclodextrin d was 3%.

A corresponding prescription amount of the cyclodextrin was dissolved with water for injection and diluted to a corresponding volume, the solution was cooled to 4ºC, the API was added and stirred until completely dissolved over about 5 minutes, and the solution was filtered with a 0.22 µm filter, diluted to 0.2 mg/mL with 5% glucose, left to stand at room temperature, and determined for 6-hour stability by means of HPLC. The data was as shown in the following table:

| | **Time (h)** | **HP1 (area%)** | **API (area%)** | **API (C/C0%)** |
|---|---|---|---|---|
| **1** | 0 | 0.25 | 99.70 | 100 |
| | 3 | 2.64 | 97.05 | 96.96 |
| | 6 | 4.82 | 94.78 | 94.53 |
| **2** | 0 | 0.24 | 99.73 | 100 |
| | 3 | 2.48 | 97.27 | 97.13 |
| | 6 | 4.40 | 95.04 | 94.74 |
| **3** | 0 | 0.22 | 99.73 | 100 |
| | 3 | 2.35 | 97.24 | 97.13 |
| | 6 | 4.31 | 95.22 | 95.04 |
| **4** | 0 | 0.21 | 99.76 | 100 |
| | 3 | 1.97 | 97.63 | 97.57 |
| | 6 | 3.49 | 96.05 | 95.68 |

It could be seen from the above table that the cyclodextrin and bendamustine at the same mass ratio showed a better stability as the chlorine content increased.

### Example 9

### Stability of compositions containing different mass proportions of PVPk12 after being diluted with 5% glucose

| | Component (each) | | API : PVPk12 |
|---|---|---|---|
| **1** | API | 100 mg | 1 : 0.5 |
| | Sulfobutyl-β-cyclodextrin | 2400 mg | |
| | PVPk12 | 50 mg | |
| | Water for injection (for dilution to a constant volume) | 4 mL | |
| **2** | API | 100 mg | 1 : 1 |
| | Sulfobutyl-β-cyclodextrin | 2400 mg | |
| | PVPk12 | 100 mg | |
| | Water for injection (for dilution to a constant volume) | 4 mL | |
| **3** | API | 100 mg | 1 : 2 |
| | Sulfobutyl-β-cyclodextrin | 2400 mg | |
| | PVPk12 | 200 mg | |
| | Water for injection (for dilution to a constant volume) | 4 mL | |

Corresponding prescription amounts of sulfobutyl-β-cyclodextrin and PVPk12 were dissolved with water for injection (the content of chlorine in sulfobutyl-β-cyclodextrin was 0.08 wt%) and diluted to a corresponding volume, the solution was then cooled to 4ºC, the API was added and stirred until completely dissolved over about 5 minutes, and the solution was filtered with a 0.22 µm filter, diluted to 0.2 mg/mL with a 5% glucose injection, left to stand at room temperature, and determined for 6-hour stability by means of HPLC. The data was as shown in the following table:

| | **Time (h)** | **HP1 (area%)** | **API (area%)** | **API (C/C0%)** |
|---|---|---|---|---|
| **1** | 0 | 0.25 | 99.55 | 100.00 |
| | 3 | 2.25 | 97.48 | 97.52 |
| | 6 | 3.96 | 95.57 | 95.54 |
| **2** | 0 | 0.23 | 99.56 | 100.00 |
| | 3 | 2.36 | 97.32 | 97.57 |
| | 6 | 4.26 | 95.23 | 95.15 |
| **3** | 0 | 0.29 | 99.42 | 100.00 |
| | 3 | 2.44 | 97.18 | 97.49 |
| | 6 | 4.33 | 95.13 | 95.48 |

### Example 10

### Stability of compositions containing PVPk12 and sodium chloride after being diluted with 5% glucose

| | Component (each) | |
|---|---|---|
| **1** | API | 100 mg |
| | Sulfobutyl-β-cyclodextrin | 2400 mg |
| | PVPk12 | 50 mg |
| | Sodium chloride | 72 mg |
| | Water for injection (for dilution to a constant volume) | 4 mL |
| **2** | API | 100 mg |
| | Sulfobutyl-β-cyclodextrin | 2400 mg |
| | PVPk12 | 50 mg |
| | Sodium chloride | 36 mg |
| | Water for injection (for dilution to a constant volume) | 4 mL |

Corresponding prescription amounts of sulfobutyl-β-cyclodextrin, PVPk12 and sodium chloride were dissolved with water for injection (the content of chlorine in sulfobutyl-β-cyclodextrin was 0.08 wt%) and diluted to a corresponding volume, the solution was then cooled to 4ºC, the API was added and stirred until completely dissolved over about 30 minutes, and the solution was filtered with a 0.22 µm filter, diluted to 0.2 mg/mL with a 5% glucose injection, left to stand at room temperature, and determined for 6-hour stability by means of HPLC. The data was as shown in the following table:

| | **Time (h)** | **HP1 (area%)** | **API (area%)** | **API (C/C0%)** |
|---|---|---|---|---|
| **1** | 0 | 0.18 | 99.80 | 100.00 |
| | 3 | 1.70 | 98.19 | 98.07 |
| | 6 | 3.11 | 96.49 | 95.65 |
| **2** | 0 | 0.21 | 99.76 | 100.00 |
| | 3 | 1.73 | 98.17 | 97.64 |
| | 6 | 3.16 | 96.44 | 95.75 |

### Example 11

### Stability at different diluted final concentrations (0.6, 1.0, 2.5, 5, 10, and 16 mg/mL)

| Component (each) | | | API : Cyclodextrin |
|---|---|---|---|
| **1** | API | 100 mg | 1 : 24 |
| | Sulfobutyl-β-cyclodextrin | 2400 mg | |
| | Water for injection (for dilution to a constant volume) | 4 mL | |

A corresponding prescription amount of sulfobutyl-β-cyclodextrin was dissolved with water for injection (the content of chlorine in sulfobutyl-β-cyclodextrin was 0.08 wt%) and diluted to a corresponding volume, the solution was then cooled to 4ºC, the API was added and stirr ed until completely dissolved over about 5 minutes, and the solution was filtered with a 0.22 µm filter, and prepared into a freeze-dried powder. The freeze-dried powder was diluted to 0.6, 1, 2.5, 5, 10, and 16 mg/mL with a 5% glucose injection, left to stand at room temperat ure, and determined for 6-hour stability by means of HPLC. The data was as shown in the fo llowing table:

| **Final concentration (mg/mL)** | **Time (h)** | **HP1 (area%)** | **API (area%)** | **API (C/C0%)** |
|---|---|---|---|---|
| **0.6** | 0 | 0.19 | 99.79 | 100.00 |
| | 3 | 1.56 | 98.30 | 97.62 |
| | 6 | 2.76 | 96.94 | 96.19 |
| **1** | 0 | 0.20 | 99.78 | 100.00 |
| | 3 | 1.27 | 98.64 | 98.98 |
| | 6 | 2.14 | 97.64 | 97.97 |
| **2.5** | 0 | 0.18 | 99.79 | 100.00 |
| | 3 | 0.84 | 99.09 | 99.49 |
| | 6 | 1.40 | 98.50 | 98.99 |
| **5** | 0 | 0.17 | 99.82 | 100.00 |
| | 3 | 0.56 | 99.41 | 100.96 |
| | 6 | 0.94 | 98.99 | 100.96 |
| **10** | 0 | 0.17 | 99.81 | 100.00 |
| | 3 | 0.41 | 99.57 | 100.00 |
| | 6 | 0.63 | 99.33 | 100.47 |
| **16** | 0 | 0.17 | 99.82 | 100.00 |
| | 3 | 0.30 | 99.69 | 100.17 |
| | 6 | 0.42 | 99.55 | 100.35 |

It could be seen from the above table that with the increase of the final concentration of dilution, the degradation rate of the API also gradually decreased at 3 h and 6 h. When taking 100-120 mg/m² body surface area (BSA) for dosagecalculation, 100-360 mg of bendamustine was needed for 1-3 m²; in addition, in the case of dilution to 500 mL, the concentration of bendamustine was 0.2-0.72 mg/mL; in the case of dilution to 250 mL, the concentration of bendamustine was 0.4-1.44 mg/mL; in the case of dilution to 100 mL, the concentration of bendamustine was 1-3.6 mg/mL; in the case of dilution to 50 mL, the concentration of bendamustine was 2-7.2 mg/mL; and in the case of dilution to 25 mL, the concentration of bendamustine was 4-14.4 mg/mL. For patients who needed a reduced infusion volume, they could all achieve a sufficient solubility at which no precipitation occurred and a stable time of not less than 3 hours (the degradation of API was not more than 5%).

### Example 12

### Reconstitution times for different reconstitution concentrations and 5% glucose dilution stability

| | Component (each) | | API : Cyclodextrin |
|---|---|---|---|
| **1** | API | 100 mg | 1 : 24 |
| | Sulfobutyl-β-cyclodextrin | 2400 mg | |
| | Water for injection (for dilution to a constant volume) | 4 mL | |

A corresponding prescription amount of sulfobutyl-β-cyclodextrin was dissolved with water for injection (the content of chlorine in sulfobutyl-β-cyclodextrin was 0.08 wt%) and diluted to a corresponding volume, the solution was then cooled to 4ºC, the API was added and stirr ed until completely dissolved over about 5 minutes, and the solution was filtered with a 0.22 µm filter, and prepared into a freeze-dried powder by means of a freeze dryer.

The freeze-drying curve was as follows: the partition board precooling temperature was 5ºC, freezing was carried out at -50ºC for 6 hours, primary drying was carried out at -20ºC for 48 hours, and secondary drying was carried out at 30ºC for 6 hours. The temperatures and times for the pre-freezing, primary drying and secondary drying could be adjusted as required.

It was reconstituted with a 5% glucose injection to 2.5, 10, and 25 mg/mL, the reconstitution time was recorded, and after reconstitution, it continued to be diluted with a 5% glucose injection to 0.2 mg/mL and was left to stand at room temperature and determined for 6-hour stability by means of HPLC. The following table showed the reconstitution time:

| **Final concentration (mg/mL)** | **Reconstitution time (sec)** |
|---|---|
| **2.5** | 30 |
| **10** | 60 |
| **25** | 180 |

The following table showed the stability data:

| **Reconstitution concentration (mg/mL)** | **Time (h)** | **HP1 (area%)** | **API (area%)** | **API (C/C0%)** |
|---|---|---|---|---|
| **2.5** | 0 | 0.29 | 99.71 | 100.00 |
| | 3 | 2.41 | 97.34 | 96.86 |
| | 6 | 4.27 | 95.41 | 94.12 |
| **10** | 0 | 0.24 | 99.76 | 100.00 |
| | 3 | 2.55 | 97.16 | 97.26 |
| | 6 | 4.64 | 94.71 | 94.52 |
| **25** | 0 | 0.21 | 99.79 | 100.00 |
| | 3 | 2.26 | 97.50 | 97.13 |
| | 6 | 4.04 | 95.14 | 94.74 |

It could be seen from the reconstitution time and dilution stability data that the larger the reconstitution volume, the shorter the reconstitution time, the reconstitution time was also far less than 15 minutes, and samples with different reconstitution volumes could all provide stability for more than 3 hours after being diluted to 0.2 mg/mL with 5% glucose.

### Example 13

### Stability of compositions with different freeze-dried volumes after being diluted with 5% glucose injection

| | Component (each) | | **Freeze-dried volume** |
|---|---|---|---|
| **1** | API | 100 mg | 12 mL |
| | Sulfobutyl-β-cyclodextrin | 2400 mg | |
| | Water for injection (for dilution to a constant volume) | 12 mL | |
| **2** | API | 100 mg | 6 mL |
| | Sulfobutyl-β-cyclodextrin | 2400 mg | |
| | Water for injection (for dilution to a constant volume) | 6 mL | |
| **3** | API | 100 mg | 4 mL |
| | Sulfobutyl-β-cyclodextrin | 2400 mg | |
| | Water for injection (for dilution to a constant volume) | 4 mL | |

A corresponding prescription amount of sulfobutyl-β-cyclodextrin was dissolved with water for injection (the content of chlorine in sulfobutyl-β-cyclodextrin was 0.08 wt%) and diluted to a corresponding volume, the solution was then cooled to 4ºC, the API was added and stirr ed until completely dissolved over about 5 minutes, and the solution was filtered with a 0.22 µm filter, and prepared into a freeze-dried powder by means of a freeze dryer. It was recon stituted with a 5% glucose injection to 25 mg/mL, and after reconstitution, it continued to be diluted with a 5% glucose injection to 0.2 mg/mL and was left to stand at room temperatur e and determined for 6-hour stability by means of HPLC. The following table showed the stab ility data:

| **Freeze-dried volume (mL)** | **Time (h)** | **HP1 (area%)** | **API (area%)** | **API (C/C0%)** |
|---|---|---|---|---|
| **12** | 0 | 0.23 | 99.77 | 100.00 |
| | 3 | 2.43 | 97.28 | 97.04 |
| | 6 | 4.33 | 95.06 | 94.58 |
| **6** | 0 | 0.27 | 99.73 | 100.00 |
| | 3 | 2.62 | 97.07 | 96.62 |
| | 6 | 4.65 | 94.66 | 93.72 |
| **4** | 0 | 0.30 | 99.70 | 100.00 |
| | 3 | 2.56 | 97.19 | 96.92 |
| | 6 | 4.48 | 94.87 | 93.99 |

It could be seen from the dilution stability data that samples with different freeze-dried volumes could all provide stability for more than 3 hours after being diluted to 0.2 mg/mL with 5% glucose.

### Example 14

### Stability of freeze-dried powders with different volumes accelerated at 40ºC for 15 days

| | Component (each) | | **Freeze-dried** |
|---|---|---|---|
| | | | **volume** |
| **1** | API | 100 mg | 12 mL |
| | Sulfobutyl-β-cyclodextrin | 2400 mg | |
| | Water for injection (for dilution to a constant volume) | 12 mL | |
| **2** | API | 100 mg | 6 mL |
| | Sulfobutyl-β-cyclodextrin | 2400 mg | |
| | Water for injection (for dilution to a constant volume) | 6 mL | |
| **3** | API | 100 mg | 4 mL |
| | Sulfobutyl-β-cyclodextrin | 2400 mg | |
| | Water for injection (for dilution to a constant volume) | 4 mL | |

A corresponding prescription amount of sulfobutyl-β-cyclodextrin was dissolved with water for injection (the content of chlorine in sulfobutyl-β-cyclodextrin was 0.08 wt%) and diluted to a corresponding volume, the solution was then cooled to 4ºC, the API was added and stirred until completely dissolved over about 5 minutes, and the solution was filtered with a 0.22 µm filter, and prepared into a freeze-dried powder by means of a freeze dryer. It was reconstituted with a 5% glucose injection to 25 mg/mL, and after reconstitution, it continued to be diluted with a 5% glucose injection to 0.2 mg/mL and was left to stand at room temperature and determined for content change by means of HPLC. The following table showed the stability data:

| | **Time (d)** | **HP1 (area%)** | **API (area%)** |
|---|---|---|---|
| **1** | 0 | 0.22 | 99.76 |
| | 15 | 0.40 | 99.51 |
| **2** | 0 | 0.21 | 99.77 |
| | 15 | 0.31 | 99.62 |
| **3** | 0 | 0.18 | 99.82 |
| | 15 | 0.30 | 99.62 |

In conjunction with the stability data, the composition was expected to provide a shelf life of not less than two years in a 2-15ºC environment

### Example 15

### Stability of API in different prescriptions 28 days after being reconstituted to 25 mg/mL

| | Component (each) | |
|---|---|---|
| **1** | API | 100 mg |
| | Sulfobutyl-β-cyclodextrin | 2400 mg |
| | Sodium chloride | 72 mg |
| | Water for injection (for dilution to a constant volume) | 4 mL |
| **2** | API | 100 mg |
| | Sulfobutyl-β-cyclodextrin | 2400 mg |
| | Sodium chloride | 72 mg |
| | PVPk12 | 50 mg |
| | Water for injection (for dilution to a constant volume) | 4 mL |
| **3** | API | 100 mg |
| | Sulfobutyl-β-cyclodextrin | 2400 mg |
| | Sodium chloride | 36 mg |
| | Water for injection (for dilution to a constant volume) | 4 mL |
| **4** | API | 100 mg |
| | Sulfobutyl-β-cyclodextrin | 2400 mg |
| | Sodium chloride | 36 mg |
| | PVPk12 | 50 mg |
| | Water for injection (for dilution to a constant volume) | 4 mL |
| **5** | API | 100 mg |
| | Sulfobutyl-β-cyclodextrin | 2400 mg |
| | Water for injection (for dilution to a constant volume) | 4 mL |

Corresponding prescription amounts of sulfobutyl-β-cyclodextrin, sodium chloride and PVPk12 were dissolved with water for injection (the content of chlorine in sulfobutyl-β-cyclodextrin was 0.08 wt%) and diluted to a corresponding volume, the solution was then cooled to 4ºC, the API was added and stirred until completely dissolved, and the solution was filtered with a 0.22 µm filter, and prepared into a freeze-dried powder by means of a freeze dryer. It was reconstituted with water for injection to 25 mg/mL, and after reconstitution, it continued to be diluted with a 5% glucose injection to 0.2 mg/mL and was left to stand at room temperature and determined for 6-hour stability by means of HPLC. The following table showed the stability data at day 0:

| | **Time (h)** | **HP1 (area%)** | **API (area%)** | **API (C/C0%)** |
|---|---|---|---|---|
| **1** | 0 | 0.22 | 99.76 | 100.00 |
| | 3 | 2.64 | 97.11 | 97.03 |
| | 6 | 4.61 | 94.80 | 94.06 |
| **2** | 0 | 0.19 | 99.77 | 100.00 |
| | 3 | 2.55 | 97.18 | 97.09 |
| | 6 | 4.57 | 94.80 | 94.17 |
| **3** | 0 | 0.20 | 99.80 | 100.00 |
| | 3 | 2.83 | 96.87 | 96.24 |
| | 6 | 5.04 | 94.24 | 92.96 |
| **4** | 0 | 0.22 | 99.76 | 100.00 |
| | 3 | 2.74 | 96.92 | 96.21 |
| | 6 | 4.81 | 94.49 | 93.36 |
| **5** | 0 | 0.24 | 99.76 | 100.00 |
| | 3 | 2.96 | 96.72 | 96.24 |
| | 6 | 5.30 | 93.92 | 92.96 |

The following table showed the stability data at day 28:

| | **Time (h)** | **HP1 (area%)** | **API (area%)** | **API (C/C0%)** | **API (C28/C0%)** |
|---|---|---|---|---|---|
| **1** | 0 | 0.42 | 99.56 | 100.00 | 100 |
| | 3 | 3.20 | 96.47 | 96.19 | |
| | 6 | 5.41 | 93.80 | 92.86 | |
| **2** | 0 | 0.43 | 99.50 | 100.00 | 99.52 |
| | 3 | 3.25 | 96.37 | 96.60 | |
| | 6 | 5.56 | 93.60 | 93.20 | |
| **3** | 0 | 0.61 | 99.36 | 100.00 | 99.53 |
| | 3 | 3.27 | 96.39 | 96.67 | |
| | 6 | 5.48 | 93.70 | 93.33 | |
| **4** | 0 | 0.59 | 99.35 | 100.00 | 99.53 |
| | 3 | 3.34 | 96.28 | 96.65 | |
| | 6 | 5.62 | 93.51 | 92.82 | |
| **5** | 0 | 1.20 | 98.75 | 100.00 | 99.07 |
| | 3 | 3.73 | 95.84 | 96.24 | |
| | 6 | 5.84 | 93.22 | 92.96 | |

It could be seen from the data that the freeze-dried powder of the composition could maintain a good stability when placed in a cold storage environment for 28 days after reconstitution, and could provide a 0.2 mg/mL 5% glucose dilution with a stable time of 3 hours, which ensured clinical use.

### Example 16

### X-ray powder diffraction of freeze-dried powder

| | Component (each) | | API : Cyclodextrin |
|---|---|---|---|
| **1** | API | 100mg | 1:24 |
| | Sulfobutyl-β-cyclodextrin | 2400mg | |
| | Water for injection (for dilution to a constant volume) | 4mL | |

A corresponding prescription amount of sulfobutyl-β-cyclodextrin was dissolved with water for injection (the content of chlorine in sulfobutyl-β-cyclodextrin was 0.08 wt%) and diluted to a corresponding volume, the solution was then cooled to 4ºC, the API was added and stirr ed until completely dissolved over about 5 minutes, and the solution was filtered with a 0.22 µm filter, and prepared into a freeze-dried powder by means of a freeze dryer, which freeze -dried powder was used for powder X-ray diffraction for 2θ angle determination. The results showed that there was no sharp crystal form characteristic peak (see Figure 2).

### Example 17

### Stability of composition diluted with 0.9% sodium chloride

| | Component (each) | | API : Cyclodextrin |
|---|---|---|---|
| **1** | API | 100 mg | 1 : 24 |
| | Sulfobutyl-β-cyclodextrin | 2400 mg | |
| | Water for injection (for dilution to a constant volume) | 4 mL | |

A corresponding prescription amount of sulfobutyl-β-cyclodextrin was dissolved with water for injection (the content of chlorine in sulfobutyl-β-cyclodextrin was 0.08 wt%) and diluted to a corresponding volume, the solution was then cooled to 4ºC, the API was added and stirr ed until completely dissolved over about 5 minutes, and the solution was filtered with a 0.22 µm filter, and prepared into a freeze-dried powder by means of a freeze dryer. It was recon stituted with normal saline to 25 mg/mL, and after reconstitution, it continued to be diluted with 0.9% sodium chloride to 0.2 and 0.6 mg/mL, and they were left to stand at room temp erature and determined for stability by means of HPLC. The following table showed the stabil ity data:

| **Concentration (mg/mL)** | **Time (h)** | **HP1 (area%)** | **API (area%)** | **API (C/C0%)** |
|---|---|---|---|---|
| **0.2** | 0 | 0.23 | 99.77 | 100.00 |
| | 3 | 1.54 | 98.39 | 98.13 |
| | 6 | 2.76 | 97.07 | 96.73 |
| | 8 | 3.52 | 96.24 | 95.79 |
| | 10 | 4.27 | 95.39 | 94.39 |
| | 12 | 4.97 | 94.59 | 93.46 |
| **0.6** | 0 | 0.21 | 99.77 | 100.00 |
| | 3 | 0.82 | 99.14 | 99.06 |
| | 6 | 1.40 | 98.52 | 98.11 |
| | 8 | 1.78 | 98.12 | 98.11 |
| | 10 | 2.14 | 97.72 | 97.17 |
| | 12 | 2.50 | 97.31 | 96.70 |

It could be seen from the above table that the stability of the bendamustine composition in 0.9% sodium chloride was significantly improved. The higher the final concentration of dilution, the better the stability.

### Example 18

### Stability of imitations of commercially available products

According to the instructions of Bendeka and Treanda, imitated preparations of the commercially available products were prepared and diluted to 0.2 mg/mL with 2.5% glucose/0.45% saline, 5% glucose and 0.9% sodium chloride, respectively, and they were placed at room temperature for stability investigation. The data was as shown in the following table:

| | **Diluent** | **Time (h)** | **HP1 (area%)** | **API (area%)** | **API (C/C0%)** |
|---|---|---|---|---|---|
| **Treanda** | 2.5% | 0 | 1.85 | 97.90 | 100.00 |
| | glucose/0.45% | | | | |
| | saline | 3 | 6.40 | 93.21 | 94.95 |
| | | 6 | 10.23 | 89.11 | 90.50 |
| | 5% glucose | 0 | 1.40 | 98.33 | 100.00 |
| | | 3 | 11.07 | 88.04 | 88.73 |
| | | 6 | 19.08 | 78.64 | 78.87 |
| | 0.9% sodium | 0 | 0.62 | 99.35 | 100.00 |
| | chloride | 3 | 5.10 | 94.70 | 94.12 |
| | | 6 | 8.95 | 90.47 | 88.24 |
| **Bendeka** | 2.5% | 0 | 0.24 | 99.46 | 100.00 |
| | glucose/0.45% | | | | |
| | saline | 3 | 4.36 | 95.31 | 95.32 |
| | | 6 | 8.07 | 91.35 | 91.12 |
| | 5% glucose | 0 | 0.47 | 99.36 | 100.00 |
| | | 3 | 10.21 | 88.98 | 88.74 |
| | | 6 | 18.21 | 79.66 | 78.83 |
| | 0.9% sodium | 0 | 0.24 | 99.74 | 100.00 |
| | chloride | 3 | 4.30 | 95.55 | 94.57 |
| | | 6 | 8.06 | 91.40 | 89.59 |

It could be seen from the above table that the existing products could not be stably compatible with 5% glucose at a concentration of 0.2 mg/mL (the degradation of API was less than 5% in 3 hours)

### Example 19

### Prescriptions with only sodium chloride and/or PVPk12

| | Component (each) | |
|---|---|---|
| **1** | API | 100 mg |
| | Sodium chloride | 72 mg |
| | Water for injection (for dilution to a constant volume) | 20 mL |
| **2** | API | 100 mg |
| | PVPk12 | 100 mg |
| | Water for injection (for dilution to a constant volume) | 20 mL |
| **3** | API | 100 mg |
| | Sodium chloride | 72 mg |
| | PVPk12 | 100 mg |
| | Water for injection (for dilution to a constant volume) | 20 mL |

A corresponding prescription amount of a stabilizer was dissolved with water for injection and diluted to a corresponding volume, the solution was cooled to 4ºC, the API was added and stirred until completely dissolved, and the solution was filtered with a 0.22 µm filter. The freeze-dried powder was diluted to 0.2 mg/mL with a 5% glucose injection, left to stand at room temperature, and determined for 6-hour stability by means of HPLC. The following table showed the stability data:

| **Freeze-dried volume** | **Time (h)** | **HP1 (area%)** | **API (area%)** | **API (C/C0%)** |
|---|---|---|---|---|
| **(mL)** | | | | |
| **1** | 0 | 0.81 | 99.19 | 100.00 |
| | 3 | 12.89 | 85.77 | 83.49 |
| | 6 | 21.35 | 74.35 | 70.36 |
| **2** | 0 | 0.75 | 99.19 | 100.00 |
| | 3 | 13.36 | 85.10 | 82.78 |
| | 6 | 21.65 | 73.89 | 69.75 |
| **3** | 0 | 0.89 | 99.07 | 100.00 |
| | 3 | 12.64 | 85.97 | 84.14 |
| | 6 | 21.13 | 74.64 | 70.80 |

It could be seen from the above table that sodium chloride and PVPk12 as stabilizers could not provide the stability that met the requirements of the present patent in the absence of a cyclodextrin.

### Example 20

### Stability of compositions containing thioglycerol after being diluted with 5% glucose injection

| | Component (each) | |
|---|---|---|
| **1** | API | 100 mg |
| | Sulfobutyl-β-cyclodextrin | 2400 mg |
| | Thioglycerol | 20 mg |
| | Water for injection (for dilution to a constant volume) | 4 mL |
| **2** | API | 100 mg |
| | Sulfobutyl-β-cyclodextrin | 2400 mg |
| | Thioglycerol | 100 mg |
| | Water for injection (for dilution to a constant volume) | 4 mL |

Corresponding prescription amounts of sulfobutyl-β-cyclodextrin and thioglycerol were dissolved with water for injection (the content of chlorine in sulfobutyl-β-cyclodextrin was 0.08 wt%) and diluted to a corresponding volume, the solution was then cooled to 4ºC, the API was added and stirred until completely dissolved over about 5 minutes, and the solution was filtered with a 0.22 µm filter, diluted to 0.2 mg/mL with a 5% glucose injection, left to stand at room temperature, and determined for 6-hour stability by means of HPLC. The following table showed the stability data:

| | **Time (h)** | **HP1 (area%)** | **API (area%)** | **API (C/C0%)** |
|---|---|---|---|---|
| **1** | 0 | 0.23 | 99.75 | 100.00 |
| | 3 | 2.55 | 97.22 | 97.03 |
| | 6 | 4.48 | 94.92 | 94.06 |
| **2** | 0 | 0.22 | 99.69 | 100.00 |
| | 3 | 3.04 | 96.61 | 96.00 |
| | 6 | 5.39 | 93.76 | 92.50 |

### Example 21

### Sodium chloride and/or glucose intakes caused by different diluents and infusion volumes

The sodium chloride and/or glucose intakes caused by different diluents and infusion volumes were calculated on the basis of a BSA of 3 and 120 mg/m2, and the results were as shown in the following table.

| | | 10 mL | 25 mL | 50 mL | 100 mL | 250 mL | 500 mL |
|---|---|---|---|---|---|---|---|
| 0.9% sodium chloride | Sodium chloride | 90 mg | 225 mg | 450 mg | 900 mg | 2250 mg | 4500 mg |
| | Glucose | - | - | - | - | - | - |
| 0.45% sodium | Sodium chloride | 45 mg | 112.5 mg | 225 mg | 450 mg | 1125 mg | 2250 mg |
| chloride | Glucose | - | - | - | - | - | - |
| Half salt and half sugar | Sodium chloride | 45 mg | 112.5 mg | 225 mg | 450 mg | 1125 mg | 2250 mg |
| | Glucose | 250 mg | 625 mg | 1250 mg | 2500 mg | 6250 mg | 12500 mg |
| 2.5% glucose | Sodium chloride | - | - | - | - | - | - |
| | Glucose | 250 mg | 625 mg | 1250 mg | 2500 mg | 6250 mg | 12500 mg |
| 5% glucose | Sodium chloride | - | - | - | - | - | - |
| | Glucose | 500 mg | 1250 mg | 2500 mg | 5000 mg | 12500 mg | 25000 mg |
| Water for injection | Sodium chloride | - | - | - | - | - | - |
| | Glucose | - | - | - | - | - | - |

It could be seen from the above table that for patients who needed reduced or eliminated sodium chloride and/or glucose, the adjustment of the different diluents and infusion volumes could well meet the needs of these patients. On the basis of a single dose of 360 mg, the mass ratio of sodium chloride as a stabilizer to bendamustine was 5 : 1, and 1800 mg of sodium chloride was the highest. At this point, after the composition was diluted with water for injection, the burden of sodium chloride was still 20-60% less than those caused by 0.9% sodium chloride, 0.45% sodium chloride, and half salt and half sugar diluents.

## Claims

1. A composition comprising the following components:
a) bendamustine or a salt thereof,
b) a cyclodextrin, and
c) an optional stabilizer,
wherein the stabilizer is selected from an inorganic or organic substance containing chlorine element, lipoic acid, thioglycerol, polyvinylpyrrolidone and any combination thereof, and
wherein the mass ratio of the bendamustine or the salt to the cyclodextrin is 1 : 5 to 1 : 100.

2. The composition of claim 1, wherein the cyclodextrin is selected from hydroxypropyl-β-cyclodextrin, sulfobutyl-β-cyclodextrin and a combination thereof.

3. The composition of claim 1 or 2, wherein the cyclodextrin is a chlorine-containing cyclodextrin, and the content of chlorine in the cyclodextrin is 0-5 wt%, preferably 0-3 wt%, and more preferably 0-1 wt%.

4. The composition of claim 1, wherein the inorganic substance containing chlorine element is selected from sodium chloride, and the organic substance containing chlorine element is selected from choline chloride.

5. The composition of claim 1, wherein the mass ratio of the bendamustine to the stabilizer is 1 : 0-5.

6. The composition of claim 1, wherein the salt of bendamustine is bendamustine hydrochloride.

7. The composition of claim 1, wherein the composition further comprises a diluent, wherein the diluent is selected from the group consisting of water for injection, 0.9% sodium chloride, 0.45% sodium chloride, 0.45% sodium chloride/2.5% glucose, 2.5% glucose and 5% glucose.

8. The composition of claim 7, wherein the diluent is 5% glucose.

9. A method for preventing or treating a cancer, the method comprising providing a subject with an injection of bendamustine or a salt thereof at a concentration of 0.2-100 mg/ml, wherein the injection comprises the bendamustine or the salt thereof, a cyclodextrin, an optional stabilizer and a diluent, with the mass ratio of the bendamustine or the salt thereof to the cyclodextrin being 1 : 5 to 1 : 100, and wherein the stabilizer is selected from an inorganic or organic substance containing chlorine element, lipoic acid, thioglycerol, polyvinylpyrrolidone and any combination thereof.

10. The method of claim 9, wherein the cancer is selected from the group consisting of chronic lymphocytic leukemia, Hodgkin's lymphoma, non-Hodgkin's lymphoma, multiple myeloma and breast cancer.

11. The method of claim 9, wherein the cyclodextrin is selected from hydroxypropyl-β-cyclodextrin, sulfobutyl-β-cyclodextrin and a combination thereof.

12. The method of claim 9 or 11, wherein the cyclodextrin is a chlorine-containing cyclodextrin, and the content of chlorine in the cyclodextrin is 0-5 wt%, preferably 0-3 wt%, and more preferably 0-1 wt%.

13. The method of claim 9, wherein the inorganic substance containing chlorine element is selected from sodium chloride, and the organic substance containing chlorine element is selected from choline chloride.

14. The method of claim 9, wherein the mass ratio of the bendamustine to the stabilizer is 1 : 0-5.

15. The method of claim 9, wherein the salt of bendamustine is bendamustine hydrochloride.

16. The method of claim 9, wherein the diluent is selected from the group consisting of water for injection, 0.9% sodium chloride, 0.45% sodium chloride, 0.45% sodium chloride/2.5% glucose, 2.5% glucose and 5% glucose.

17. The method of claim 9, wherein the concentration of the bendamustine or the salt thereof is 0.2-16 mg/mL.

18. The method of claim 16, wherein the diluent is a 5% glucose injection.

19. Use of the composition of any one of claims 1-8 in the preparation of a medicament for treating a cancer.

20. The use of claim 19, wherein the cancer is selected from the group consisting of chronic lymphocytic leukemia, Hodgkin's lymphoma, non-Hodgkin's lymphoma, multiple myeloma and breast cancer.
